# EUROPEAN PATENT APPLICATION

(11) **EP 4 275 487 A1**
(43) Date of publication of application: **15.11.2023**
(21) Application number: 22172402.4
(22) Date of filing: 09.05.2022
(51) Int. Cl.: A01K 67/033, B07C 5/36

(54) **INSECT SORTING DEVICE FOR THE SORTING OF LIVE INSECTS, HANDLING UNIT FOR HANDLING INSECTS COMPRISING AN INSECT SORTING DEVICE AND FARM FOR REARING INSECTS**

(71) Applicant: Bühler AG, 9240 Uzwil (CH)
(72) Inventor: WOODING, Vaughan, 9230 Flawil (CH)
(74) Representative: IPrime Künsch Patentanwälte GmbH

(57) **Abstract**

The invention refers to an insect sorting device (21) for the sorting of live insects (12). The insect sorting device (21) comprising a conveyor (26) which comprises a movable section (27) having a first end (28) and a second end (29) and a drive means (36) for moving the moveable section (27) from the first end (28) to the second end (29), a separating device (41) for taking away the live insects (12) from the conveyor (26), a live insect collecting device (46) for collecting the live insects (12), and a dead insect collecting device (51) for collecting the dead insects (13). In use of the insect sorting device (21) the second end (29) of the movable section (27) is arranged higher in respect to a horizontal plane than the first end (28) of that movable section (27).

## Description

The present invention refers to an insect sorting device for the sorting of live insects according to the preamble of claim 1, a handling unit for handling insects comprising such an insect sorting device according to claim 14 as well as a farm for rearing insects according to claim 15.

### Technological Background

Growing and rearing insect species comes with unique challenges such as separating live and dead insects from one another. Having dead insects return into the rearing or breed process is inefficient and unhygienic. When producing insect products for food or feed applications it is also necessary to only put through live insects into the processing line as dead insects can lead to health and quality risks. Moreover, the insects are mostly mixed with frass which is also not favourable in further processing and therefore has also to be separated out.

US20180070566A1 discloses a farm for rearing insects having a rearing zone and a handling zone. For sorting out of insects which died during rearing air sifters may be used.

KR10174928B1 discloses an insect sorting device comprising a horizontal arranged conveyor for transporting larvae. Dead insects and frass fall over the second end of the conveyor.

The disadvantage of these solutions is that the quality of sorting out of the live insects is unsatisfactory for an industrial use.

JPH092412A discloses an insect sorting device comprising a horizontal arranged conveyor for transporting larvae. A size selection camera and a body colour determination camera as well as two sorting air nozzles are provided. Depending on the identified size and colour the larvae are sorted out by the blowing air. Dead larvae and frass will be collected in a waste container provided at the end of the conveyor.

The disadvantage of this solution is that for a satisfactory sorting out the device must be of a complex embodiment.

WO2021233945A1 discloses a device for sorting insects, having a belt conveyor with an upper surface and a lower surface formed by the belt of the conveyor. When the belt has been turned over to pass over the lower surface of the conveyor, all or some of the insects of a mixture deposited thereon remain attached to the belt, while the rest of the mixture fall under gravity. While the upper surface of the conveyor is substantially flat and horizontal along the majority of its length between the first end and the second end, it has an end portion, adjacent to the second end, having a downward inclination of between 20° and 90° with respect to the rest of the upper surface of the conveyor.

The disadvantage of this solution is that the quality of sorting out of the live insects is still unsatisfactory for an industrial use.

### Explanation of the Invention

Aim of the invention is to provide an insect sorting device which does not have at least one of the aforementioned disadvantages and which especially guarantees a perfect sorting out of live insects, dead insects, and frass.

These objects of the invention are at least partly solved by the features of the independent claims. Preferred developments of the invention are mentioned in the dependent claims.

The inventive insect sorting device for the sorting of live insects comprising a conveyor which comprises at least one movable section for transporting the insects, wherein the at least one movable section having a first end and a second end, and a drive means for moving the at least one moveable section from the first end to the second end, a separating device for taking away the live insects from the conveyor, a live insect collecting device for collecting the live insects sorted out and a dead insect collecting device for collecting the dead insects sorted out. In use of the inventive insect sorting device the second end of the at least one movable section is arranged higher in respect to a horizontal plane than the first end of that movable section.

The at least one moveable section of the conveyor is at least partly moved upwards which provides an urgency for the live insect to hold on that moveable section. Dead insects and frass slide downwards due to the gravity forces acting on the mixture provided on the conveyor.

The live insect collecting device and/or the dead insect collecting device may be crates or boxes. Alternatively, these collecting devices may - as non-exhaustive examples - be another conveyor, a bucket, or a silo.

The conveyor may be at least a part of a transporting means for transporting at least the live insects.

The invention provides an easily constructible device wherein the loss of live insects is low and reduced in comparison with the solutions according to the prior art. Moreover, with the invention a high degree of sorting out will be provided. Thereby, the inventive insect sorting device can be integrated as an automated system whereby no operator or human interaction is necessary. Moreover, the inventive insect sorting device is usable for different kinds of insects, especially non-flying adult insects or larval stage insects, and for adults, larvae pre-pupae or different insect life stages.

The live insects separated by the insect separating device are further processed or are return to breeding.

Advantageously, the majority of (or most of) the length of the movable section is different from a horizontal arrangement that the main part of the conveyor provides a slide for the dead insects and frass and the effect of gravity is better used.

Preferred, the movable section of the conveyor is arranged at least partly inclined, whereby the effect of gravity is used for separating live insects from dead insects and frass.

Preferably, the angle between an inclined movable section of the conveyor and a horizontal plane exceeds at least 10°, preferably exceeds at least 15°, such that the sliding of dead insects and frass along the inclined movable will be improved without further means that support the sliding. Mainly depending on the kind of the at least movable section the angle between the inclined movable section of the conveyor and a horizontal plane exceeds more than 30°, that the sliding of dead insects and frass along the inclined movable will be further improved. However, the angle is preferably chosen based on the capability for the live insects to still hold on.

Advantageously, the inclination of the movable section of the conveyor changes over the length of the conveyor, that different forces acting on the mixture whereby the separation of live insects from dead insects and frass is further improved.

Preferably, the angle of the inclined movable section of the conveyor is adjustable that the insect sorting device can be adjusted depending on the kind and/or stage of maturity of the live insects to guarantee a high level of separation. Further preferred, the inclination of the movable section of the conveyor is dynamically changeable which further improves the separation of live insects from dead insects and frass.

Preferred the separating device comprising a scraper means for scraping off the live insects from the conveyor that the live insects are easily collectable at a defined position of the conveyor. The scraper means may be - as non-exhaustive examples - a knife, a brush, or a barrier element.

The separating device for separating the live insects from the conveyor may be either a stationary device such, e.g., a fixed installed knife or brush, or a dynamic device, e.g., a rotary brush. Furthermore, this separating device may comprise an air separator which provides an air jet, a high-power air jet or suction or pulsed movement on the movable section of the conveyor.

Alternatively or additionally, the separating device for separating the live insects from the conveyor may be configured to stimulate the live insects that the live insects are released from the movable section of the conveyor. Such a stimulation may be provided by an electric shock, whereby this electrical shock preferably be based on low level electricity, and/or by a sudden temperature change. The stimulation is of such a kind that the live insects are not harmed by this procedure.

A further separating device may be provided for cleaning the movable section of the conveyor, especially from frass sticking on the movable section of the conveyor. Such a further separating device may be a brush.

Advantageously, the movable section of the conveyor comprising at least one conveyor belt, which runs around the first end and the second end. Alternatively, the conveyor comprising at least one conveyor band. The movable section of the conveyor may be formed by two or more conveyor belts or conveyor bands which are arranged one after another, preferably in a row.

Preferably the at least one conveyor belt or conveyor band is porous and/or provided with holding means for the live insects to hold on. Such a conveyor belt or conveyor band provides a high friction that allows the live insects to securely grab the belt. The holding means are apart from other embodiments knobs, strands, and/or hooks.

Preferred, the at least one conveyor belt or conveyor band is twisted at least partly along the length of the at least one conveyor belt or conveyor band that a high grade of separation is provided. Live insects will still hold on the at least one twisted conveyor belt or conveyor band whereby the dead insects and frass will be falling from the at least one twisted conveyor belt or conveyor band.

Advantageously, a shaking means and/or vibrating means is provided for shaking and/or vibrating at least the movable section of the conveyor what additionally support the separation of live insects from dead insects and frass. Furthermore, shaking and/or vibrating may ensure an even distribution of the mixture or insects.

Alternatively, the movable section of the conveyor is formed as a drum in which the mixture is provided. The live insects grab inside of the drum the movable section and dead insects as well as frass fall and can be removed.

Preferred, an air separator is provided for separating the live and/or dead insects on the conveyor by air whereby the separation is easily done with a high separation value.

Preferably, the air separator is arranged in the region of the second end of the conveyor thereby the necessary force of the air stream can be reduced to a necessary minimum what results in an economically solution of the insect sorting device whereby the separation is still on a high separation value. If the movable section is a belt or band, the air separator is preferably arranged for a very effective separation shortly before the turnaround of the belt or band.

Advantageously, the air separator is a blower device and/or a suction device that according to the respective requirements a blowing force and/or a suction force can be provided to support the separation of live insects from dead insects and frass.

The blowing force and/or the suction force may support
- removing dead insects and frass. Therefore, the respective blowing force and/or suction force can be provided at any point along the movable section of the conveyor;
- encouraging live insects to grab the surface of the movable section of the conveyor. The respective blowing force and/or suction force can be provided at any point along the movable section of the conveyor;
- removing live insects from the movable section of the conveyor. The respective blowing force and/or suction force may be provided most likely in the region of the second end of the movable section of the conveyor. Preferably, the blowing force and/or suction force is of high pressure. The value of the pressure provided mainly depends on the holding method provided for the insects;
- cleaning of the movable section of the conveyor. Such a cleaning is especially very effective, if the movable section of the conveyor is porous, e.g., a porous belt or band.

Preferably, the air separator is designed to provide or suck the air in a pulsed or constant way whereby the separation may be easily adjusted at the respective requirements and/or mixture and/or kind and mature of the insects.

The air separator may also be arranged on the underside of the movable section of the conveyor, especially if the movable section of the conveyor is a belt or band. The live insects may pass the turnaround and then will be separated by the air separator.

Preferred, the air separator as a blower device is designed to blow air through the movable section of the conveyor and/or a suction device is designed to suck air through the movable section of the conveyor what especially support cleaning of the movable section of the conveyor.

Advantageously, two or multiple conveyors are provided whereby the finally separated live insects are mainly released from any dead insects and frass by multiple separation steps. This would be a preferably solution, e.g., if the live insects are further processed for feed or food.

Preferred, at least one sensing device is provided for sensing a respective value. The sensing device may sense, e.g., vitality, weight, density, color, or other values. The sensing devices may be an electrical device, an electronical device, an infrared device, an optical device, and/or a mechanical device, selected respective the value that must be sensed. Preferably, non-contact type sensors are used. With at least one sensing devices easily can determined a good quality of the live insects or the status of the live insects as such, e.g., no discoloration, fully intact insect (shape), heat, etc..

The inventive handling unit for handling insects comprising such an insect sorting device which comprising at least a part of the aforementioned features allows an effective handling also in an industrial scale. The live insects separated by the insect separating device are further processed or are returned to breeding.

The inventive farm for rearing insects comprising at least one rearing zone for rearing insects and at least one insect sorting device which comprising at least a part of the aforementioned features. Such a farm allows an effective rearing in an industrial scale.

### Explanation of the Invention

By means of the following figures, the invention is explained in more detail with the aid of examples of embodiments. The list of reference signs is part of the disclosure.

Positional indications such as "up", "above", "down", "below", "right" or "left" are in each case related to the corresponding figures and are not to be understood as restrictive.

The figures are described coherently and comprehensively. Identical reference signs mean identical components. It shows
- Fig. 1: a first embodiment of the inventive insect sorting device in a schematic side view;
- Fig. 2: a second embodiment of the inventive insect sorting device in a schematic side view;
- Fig. 3: a third embodiment of the inventive insect sorting device in a schematic side view;
- Fig. 4: a fourth embodiment of the inventive insect sorting device in a schematic side view;
- Fig. 5: a fifth embodiment of the inventive insect sorting device in a perspective view;
- Fig. 6: a sixth embodiment of the inventive insect sorting device in a perspective view;
- Fig. 7: a seventh embodiment of the inventive insect sorting device in a perspective view;
- Fig. 8: a farm for rearing insects comprising a handling unit in schematic plan view.

### Description of the Figures

In the following the invention is described in connection with mealworms (e.g., tenebrio molitor, zophobas morio, alphitobius diaperinus). The invention is not restricted to mealworms only. It can also be used for other kind of insects, as, e.g., crickets, grasshoppers, cockroaches, ants, termites, etc.

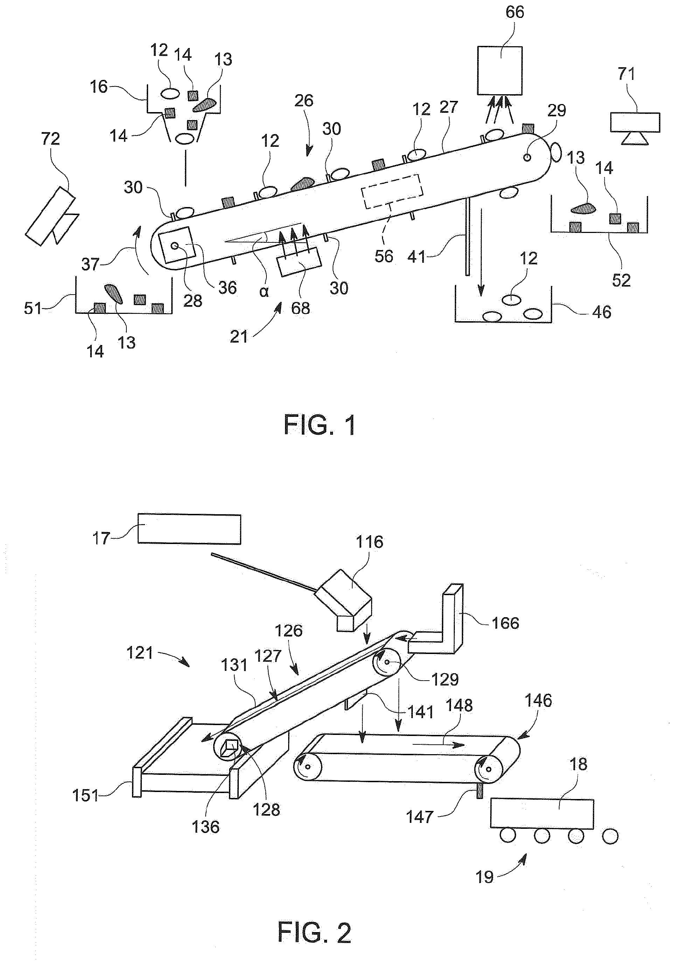

The insect sorting device 21 as shown by Fig. 1 comprises a conveyor 26. The conveyor 26 comprises a movable section 27 for transporting the insects 12. The movable section 27 is a conveyor belt running around a first end 28 and around a second end 29. In use of the insect sorting device 21 the second end 29 of the movable section 27 is arranged higher in respect to a horizontal plane than the first end 28 of the movable section 27. A mixture 11 comprising live insects 12, dead insects 13 and frass 14 is provided by a funnel-shaped input device 16 on the movable section 27 near its first end 28. The conveyor belt is provided with holding means 30 for the live insects 12 to hold on and may also be porous for providing a better grip for the live insects 12.

A motor is provided as a drive means 36 for moving the moveable section 27 upwards from the first end 28 to the second end 29 (see arrow 37) and back. The movable section 27 of the conveyor 26 is arranged inclined and therefore the arrangement of the movable section 27 of the conveyor 26 is different from a horizontal arrangement. The angle α between the inclined movable section 27 of the conveyor 27 and a horizontal plane exceeds 15°. An angle adjustment mechanism may be provided for adjusting the angle α of the inclined movable section 27 of the conveyor 26. Preferably, such an angle adjustment mechanism is configured for dynamically changing the angle α.

A separating device 41 comprises a scraper means, in this example a blade, which is provided underneath of the movable section 27 of the conveyor 26 for scraping off the live insects 12 from the conveyor 26 which still gripping on the conveyor belt. A crate as a live insect collecting device 46 is provided under the separating device 41 and the second end 29 of the movable section 27 of the conveyor 26. The live insects 12 taken away from the conveyor 26 fall into the live insect collecting device 46.

A first dead insect collecting device 51 in form of a crate is provided at the first end 28 of the conveyor 26. Parts of the mixture 11, namely frass 14 and dead insects 13, which slide downwards the movable section 27 of the conveyor 26 are collected in the first dead insect collecting device 51.

A second dead insect collecting device 52 in form of a crate is provided at the second end 29 of the conveyor 26. Parts of the mixture 11, namely frass 14 and dead insects 13 which were transported upwards fall over the second end 29 of the conveyor 26 and are collected in the second dead insect collecting device 52.

The content of the first dead insect collecting device 51 and/or of the second dead insect collecting device 52 may be provided again on the conveyor 26 to separate live insects 12 which may be still present therein.

A vibrating means 56 may be provided for vibrating at least the movable section 27 of the conveyor 26 that the live insects 12 are further urged to securely grip the movable section 27 of the conveyor 26. Instead of the vibrating means 56 or additionally to that, a shaking means may be provided for shaking at least the movable section 27 of the conveyor 26. Usable vibrating means 56 and/or shaking means for vibrating and/or shaking at least the movable section 27 of the conveyor 26 are well known on the market.

A suction device as an air separator 66 is provided near of the second end 29 of the movable section 27 of the conveyor. At least a part of the frass 14 and/or the dead insects 13 may be sucked in by the air separator 66. To improve the separation the air stream provided may be pulsed. Alternatively, the air stream is provided in a constant way.

The content sucked in by the air separator 66 may be provided again on the conveyor 26 to separate live insects 12 which may be still present therein.

A further air separator 68 is provided underneath the movable section 27 of the conveyor 26. This further air separator 68 is a blower device and is designed to blow air through the movable section 27 of the conveyor 26. Alternatively or additionally, the further air separator 68 may be a suction device and may designed to suck air through the movable section 27 of the conveyor 26.

Different sensing devices may be provided for sensing respective values. The sensing devices are direct connected, or wireless connected with a control of the insect sorting device 21. As non-exhaustive examples, cameras 71 and 72 may be provided as sensing devices for sensing possible live insects 12 in the dead insect collecting devices 51 and 52. Based on the sensed values sensed by the cameras the velocity of the conveyor may be adjusted. The drive means 36 may be provided with a torque sensor for providing respective values for the control of the insect sorting device 21.

In comparison to the aforementioned embodiment described in relation to Fig. 1, the insect sorting device 121 according to Fig. 2 comprises an input device 116 which is arranged near the second end 129 of the movable section 127 of the conveyor 126. A mixture from a rearing crate 17 is introduced into the input device 116 and is provided on the movable section 127 (here a conveyor belt) of the conveyor 126. The mixture provided slides along the movable section 127 to the first end 128 of the movable section 127 of the conveyor 126 (along arrow 131). Live insects will grab on the movable section 127 of the conveyor 126, whereby frass and dead insects will be collected in the dead insect collecting device 151. The sliding of the mixture into direction of arrow 131 is here supported by an air separator 166 in form of a blower device which blows air over the second end 129 of the movable section 127 of the conveyor 126.

The drive means (e.g., a hydraulic motor) moves the movable section 127 of the conveyor 126 upwards to the second end 128 of the movable section 127 of the conveyor 126. Live insects will be scraped off from the conveyor 126 by the scraper means of the separating device 141 and fall onto the live insect collecting device 146, which is in form of a further conveyor. The live insects will afterwards be transported by this live insect collecting device 146 in direction of the arrow 148 to a crate 18 which afterwards will be transported away by a respective transporter device 19. For scraping off live insects still grabbing on the conveyor belt of the further conveyor (live insect collecting device 146) a separating device 147, e.g., in form of a brush, is arranged underneath of this further conveyor.

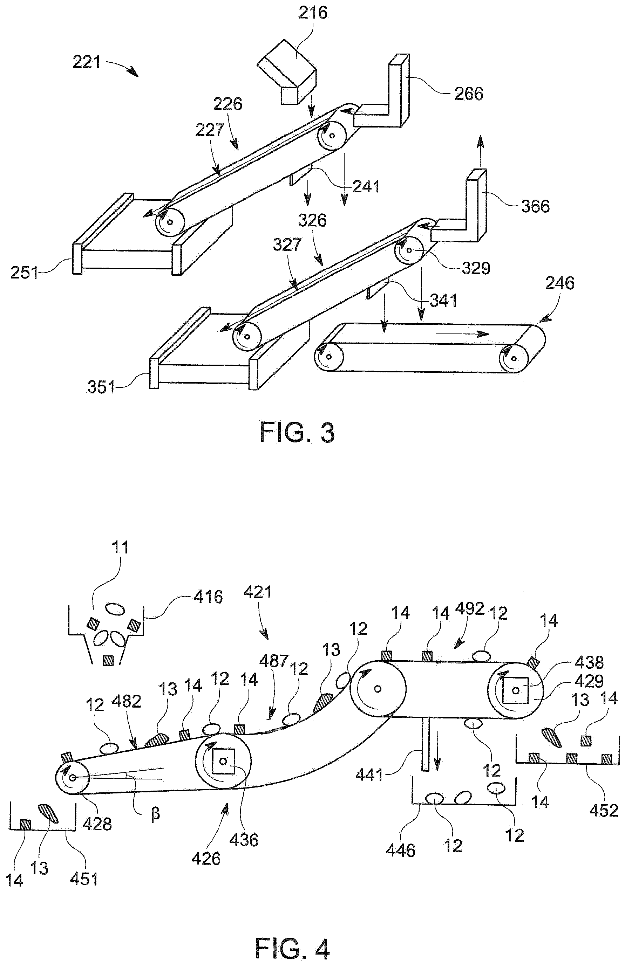

The insect sorting device 221 according to Fig. 3 provides two separation steps and comprises a first conveyor 226 and underneath arranged of this first conveyor 226 a second conveyor 326. Each movable section 227 and 327 is moved upwards.

The mixture provided by the input device 216 falls on the first conveyor 226. Supported by the blower device as an air separator 266 frass and dead insects slide downwards the conveyor 226 into first dead insect collecting device 251. Live insects and possible rest of frass and dead insects fall onto the second conveyor 326. Also, live insect scrapped away by the separating device 241 falls onto the second conveyor 326.

The air separator 366 is provided as a suction device near the second end 329 of the movable section 327 of the conveyor 326 and sucks frass as well as dead insects away from the conveyor 326. Frass and dead insects slide downwards the second conveyor 326 into second dead insect collecting device 351. Live insects fall due the gravity or by the separation device 341 on the live insect collecting device 246, also designed as a conveyor.

Further conveyors for separation may be provided that further multiple separation steps can take place for higher level of separation.

In the embodiment of an insect sorting device 421 according to Fig. 4 the conveyor 426 for moving the live insects 12 upwards comprises three movable sections 481, 486 and 491 which are interconnected to each other in a row. Two drive means 436 and 438 are provided for moving the movable sections 482, 487 and 492, each in a form of a conveyor belt, of the conveyor 426.

The first **movable section 482 of the conveyor 426 is arranged inclined wherein the angle β is** at least 10° to a horizontal plane. The second movable section 487 of the conveyor 426 runs in a bow upwards. The third movable section 492 of the conveyor 426 is arranged horizontal. The majority of the length of all movable sections 481, 486 and 491 of the conveyor 426 is different from a horizontal arrangement.

The mixture 11 is provided by the input device 416 near the first end 428 of the first movable section 482 of the conveyor 426. A part of the frass 14 and dead insects 13 fall into the dead collecting device 451 provided underneath the first end 428 of the first movable section 482 of the conveyor 426. Live insects 12 grab on the conveyor and are transported with the rest of frass 14 and dead insects 13 upwards. At the second end 429 of the conveyor 426 frass 14 and dead insects 13 falls into the second dead insects collecting device 452 due the gravity forces. Live insects fall into the live insects collecting device 446, maybe scrapped away from the conveyor 426 with the separating device 441.

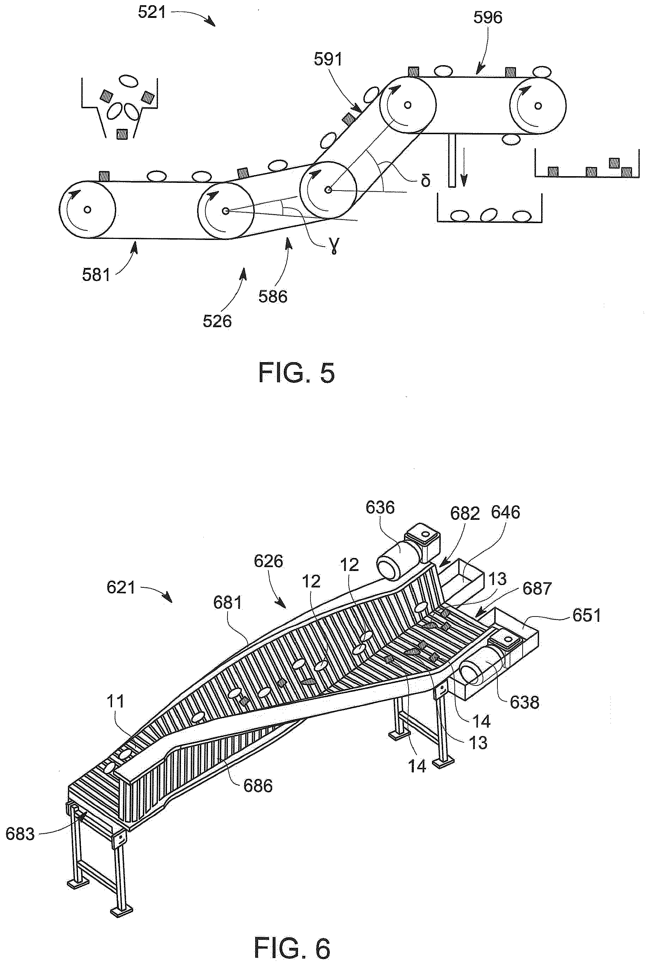

The insect sorting device 521 (shown in Fig. 5) comprises a conveyor 526 which comprises four movable sections 581, 586, 591 and 596 which are interconnected to each other in a row. In this example the first movable sections 581 of the conveyor 526 is arranged horizontal. The **second movable sections 586 of the conveyor 526 is inclined in a first angle γ which in respect** to a horizontal plane is 20°. The third movable sections 591 of the conveyor 526 is also **inclined, but in a second angle δ which in respect to a horizontal plane** is 45°. The fourth movable sections 596 of the conveyor 526 is arranged horizontal. The second movable sections 586 and the third movable sections 591 of the conveyor 526 define the majority of the length of the conveyor 526.

According to Fig. 6 the insect sorting device 621 comprises a conveyor 626 having two movable sections 681 and 686. Both movable sections 681 and 686 of the conveyor 626 are formed as conveyor belts which are twisted along the length of the conveyor belt and are arranged beneath each other. The first movable section 681 of the conveyor 626 starts horizontal arranged and changes to its second end 682 to a vertical arrangement. For moving the first movable section 681 of the conveyor 626 a first drive means 636, e.g., an electrical motor, is provided at the second end 682. The second movable section 686 of the conveyor 626 starts vertical arranged and changes to its second end 687 to a horizontal arrangement. For moving the second movable section 686 of the conveyor 626 a second drive means 638, e.g., an electrical motor, is provided at the second end 687. The first and the second drive means 636 and 638 may be synchronized.

The mixture 11 is provided on the first end 683 of the first movable section 681 of the conveyor 626. At least when the first movable section 681 changes its orientation the live insects 12 will grab on the first movable section 681. Frass 14 and dead insects 13 fall onto the second movable section 686 which slightly changes its orientation to horizontal. At the second end 682 of the first movable section 681 of the conveyor 626 the live insects 12 fall into the live insects collecting device 646. A separating device may be arranged (not shown) for scrapping off live insects 12 still grabbing onto the first movable section 681 of the conveyor 626. The frass 14 and the dead insects fall into the dead insects collecting device 651 provided at the second end 687 of the second movable section 686 of the conveyor 626.

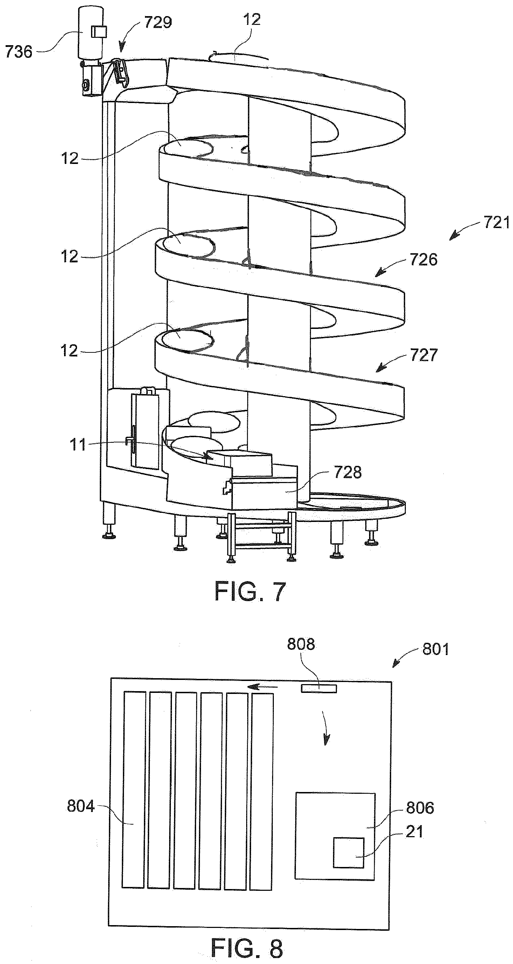

The insect sorting device 721 (shown by Fig. 7) comprises a movable section 727 which runs helical upwards driven by the drive means 736 provided at the second end 729 of the movable section 727. The mixture from live insects 12, frass and dead insects will be provided near the first end 728 of the movable section 727 of the conveyor 726. The live insects 12 are collected at the second end 729 of the movable section 727 of the conveyor 726. Frass and dead insects are collected at the first end 728 of the movable section 727 of the conveyor 726.

The rearing farm 801 as shown in Fig. 8 may be a hall with a rearing zone 804 and a handling unit 806 which comprises an insect sorting device, e.g., 21, having at least some of the aforementioned features. According to the process provided in the rearing farm 801 the respective embodiment of the inventive insect sorting device is selected.

The handling unit 806 may comprises further handling devices, as, e.g., washing station(s), tipping device(s), transporting device(s), sensing device(s), whereby this listing is non-exhaustive.

In the rearing zone 804 the insects are arranged in crates, which are mostly stapled and arranged in rows. After a defined maturing of the insects, the crates are moved out from the rearing zone 804 to the handling unit 806 by respective transport means 808. These crates contain apart from live insects also frass and dead insects.

For sorting out of the live insects an inventive insect sorting device, e.g., 21, is used. The thereby collected live insects are directly used for food or feed production. If the maturing stage of the collected live insects is not at the requested level, the collected live insects will be placed in new prepared crates and will be returned in the rearing zone 804 by the transport means 808 for further maturing.

### List of Reference Signs

- 11: mixture
- 12: live insect
- 13: dead insect
- 14: frass

- 16: input device
- 17: rearing crate
- 18: transport crate
- 19: transporter device

- 21: insect sorting device

- 26: conveyor
- 27: movable section
- 28: 1st end of 27
- 29: 2nd end of 27
- 30: holding means

- 36: drive means
- 37: arrow

- 41: separating device

- 46: live insect collecting device

- 51: 1st dead insect collecting device
- 52: 2nd dead insect collecting device

- 56: vibrating means
- 66: air separator (suck)

- 68: air separator (blow)

- 71: camera
- 72: camera

- 116: input device

- 121: insect sorting device

- 126: conveyor
- 127: movable section
- 128: 1st end of 127
- 129: 2nd end of 127

- 131: arrow

- 141: separating device

- 146: live insect collecting device
- 147: separating device

- 148: arrow

- 166: air separator (suck)

- 221: insect sorting device
- 226: 1st conveyor
- 227: movable section

- 241: separating device

- 246: live insect collecting device

- 251: 1st dead insect collecting device

- 266: air separator (blow)

- 326: 2nd conveyor
- 327: movable section

- 329: 2nd end of 327

- 341: separating device

- 351: 2nd dead insect collecting device

- 366: air separator (suck)

- 421: insect sorting device

- 426: conveyor

- 428: 1st end of 27
- 429: 2nd end of 27

- 441: separating device

- 446: live insect collecting device
- 451: 1st dead insect collecting device
- 452: 2nd dead insect collecting device

- 482: 1st movable section

- 487: 2nd movable section

- 492: 3rd movable section

- 521: insect sorting device

- 526: conveyor

- 581: 1st movable section

- 586: 2nd movable section

- 591: 3rd movable section

- 596: 4th movable section

- 621: insect sorting device

- 626: conveyor

- 636: 1st drive means

- 638: 2nd drive means

- 646: live insects collecting device
- 651: dead insects collecting device

- 681: 1st movable section
- 682: 2nd end of 681
- 683: 1st end of 681

- 686: 2nd movable section
- 687: 2nd end of 686

- 721: insect sorting device

- 727: movable section
- 728: 1st end of 727
- 729: 2nd end of 727

- 736: drive means

- 801: rearing farm

- 804: rearing zone

- 806: handling unit

- 808: transport means

## Claims

1. Insect sorting device for the sorting of live insects (12) comprising
a conveyor (26; 126; 226, 326; 426; 526; 626; 726) which comprises
at least one movable section (27; 127; 227, 327; 482, 487, 492; 581, 586, 591, 596; 681, 686; 727) for transporting the insects, wherein the at least one movable section (27; 127; 227, 327; 482, 487, 492; 581, 586, 591, 596; 681, 686; 727) having a first end (28; 128; 428; 683; 728) and a second end (29; 129; 329; 429; 682, 687; 729), and
a drive means (36; 136; 436, 438; 636, 638; 736) for moving the at least one moveable section (27; 127; 227, 327; 482, 487, 492; 581, 586, 591, 596; 681, 686; 727) from the first end (28; 128; 428; 683; 728) to the second end (29; 129; 329; 429; 682, 687; 729),
a separating device (41; 141; 241, 341; 441) for taking away the live insects (12) from the conveyor (26; 126; 226, 326; 426; 526; 626; 726),
a live insect collecting device (46; 146; 246; 446; 646) for collecting the live insects (12) sorted out, and
a dead insect collecting device (51, 52; 151; 251, 351; 451, 452; 651) for collecting the dead insects (13) sorted out, **characterized in that**
in use of the insect sorting device (21; 121; 221; 421; 521; 621; 721) the second end (29; 129; 329; 429; 682, 687; 729) of at least one movable section (27; 127; 227, 327; 482, 487; 586, 591; 681; 727) of the conveyor (26; 126; 226, 326; 426; 526; 626; 726) is arranged higher in respect to a horizontal plane than the first end (28; 128; 428; 683; 728) of that movable section (27; 127; 227, 327; 482, 487; 591; 681; 727) of the conveyor (26; 126; 226, 326; 426; 526; 626; 726).

2. Insect sorting device according to claim 1, **characterized in that** the majority of the length of the movable section (27; 127; 227, 327; 482, 487; 586, 591; 681, 686; 727) of the conveyor (26; 126; 226, 326; 426; 526; 626; 726) is different from a horizontal arrangement.

3. Insect sorting device according to claim 1 or 2, **characterized in that** the movable section (27; 127; 227, 327; 482; 586, 591; 727) of the conveyor (26; 126; 226, 326; 426; 526; 726) is arranged at least partly inclined,
whereby preferably the angle (α; β; δ, γ) between an inclined movable section (27; 127; 227, 327; 482; 586, 591; 727) of the conveyor (26; 126; 226, 326; 426; 526; 726) and a horizontal plane exceeds at least 10°, preferably exceeds at least 15,
whereby further preferably the angle (α; β; δ, γ) of the inclined movable section (27; 127; 227, 327; 482; 586, 591; 727) of the conveyor (26; 126; 226, 326; 426; 526; 726) is adjustable, preferably dynamically changeable.

4. Insect sorting device according to claim 3, **characterized in that** the inclination of the movable section (482, 487; 586, 591) of the conveyor (426; 526) changes over the length of the conveyor (426; 526).

5. Insect sorting device according to any of claims 1 to 4, **characterized in that** the separating device (41; 141; 241, 341; 441) comprising a scraper means for scraping off the insects from the conveyor.

6. Insect sorting device according to any of claims 1 to 5, **characterized in that** the movable section (27; 127; 227, 327; 482, 487, 492; 581, 586, 591, 596; 681, 686) of the conveyor (26; 126; 226, 326; 426; 526) comprising at least one conveyor belt or conveyor band, whereby preferably the at least one conveyor belt or conveyor band is porous and/or provided with holding means (30) for the live insects (12) to hold on.

7. Insect sorting device according to claim 6, **characterized in that** the at least one conveyor belt (681, 686) or conveyor band is twisted at least partly along the length of the at least one conveyor belt.

8. Insect sorting device according to any of claims 1 to 7, **characterized in that** a shaking means or vibrating means (56) is provided for shaking or vibrating at least the movable section (27) of the conveyor (26).

9. Insect sorting device according to any of claims 1 to 8, **characterized in that** an air separator (66; 166; 266, 366) is provided for separating the live insects (12) and/or dead insects (13) on the conveyor (26; 126; 226, 326) by air,
whereby preferably the air separator (66; 166; 266, 366) is arranged in the region of the second end (29; 129; 329) of the conveyor (26; 126; 226, 326).

10. Insect sorting device according to claim 9, **characterized in that** the air separator (66; 166; 266, 366) is a blower device and/or a suction device and
preferably designed to provide or suck the air in a pulsed or constant way.

11. Insect sorting device according to claim 9 or 10, **characterized in that** the air separator (68) as a blower device is designed to blow air through the movable section (27) of the conveyor (26) and/or a suction device is designed to suck air through the movable section of the conveyor.

12. Insect sorting device according to any of claims 1 to 11, **characterized in that** two or multiple conveyors (226, 326) are provided.

13. Insect sorting device according to any of claims 1 to 12, **characterized in that** at least one sensing device (71, 72) is provided.

14. Handling unit (806) for handling insects (12) comprising at least one insect sorting device (21; 121; 221; 421; 521; 621; 721) according to any of claims 1 to 13.

15. Rearing farm (801) for rearing insects (12) comprising at least one rearing zone (804) for rearing insects (12) and at least one insect sorting device (21; 121; 221; 421; 521; 621; 721) according to any of claims 1 to 13.
